# EUROPEAN PATENT SPECIFICATION

(11) **EP 0 522 669 B1**
(45) Date of publication and mention of the grant of the patent: **06.04.2005**
(21) Application number: 92203090.3
(22) Date of filing: 15.10.1990
(51) Int. Cl.: B01J 23/80, C07C 29/149

(54) **Process for preparing hydrogenation catalysts**
Verfahren zur Herstellung von Hydrierungskatalysatoren
Procédé de préparation de catalyseurs d'hydrogénation

(30) Priority: 17.10.1989 US 422624
(43) Date of publication of application: 13.01.1993
(62) Divisional of application: 90311264.7
(73) Proprietor: ENGELHARD CORPORATION, Iselin, New Jersey 08830-0770 (US)
(72) Inventor: Thakur, Deepak S., Solon, Ohio 44139 (US); Sullivan, Thomas J., Strongsville, Ohio 44136 (US); Roberts, Brian D., Solon, Ohio 44139 (US); Vlchek, Anita L., Mentor, Ohio 44060 (US)
(74) Representative: Fisher, Adrian John

(56) References cited:
- EP-A- 0 109 703
- EP-A- 0 146 165
- EP-A- 0 152 314
- DE-A- 3 717 111

## Description

### Technical Field

This invention relates to a process for preparing curtain catalysts which are particularly useful as hydrogenation catalysts, and more particularly, as catalysts for hydrogenating aldehydes, ketones, carboxylic acids and carboxylic esters. The present invention relates especially to a process for preparing such catalysts which are useful in hydrogenation reactions and which comprise the oxides of copper, zinc and aluminium.

### Background of the Invention

The preparation of various copper-containing catalysts and the use of such catalysts in various reactions has been described previously. Such reactions include hydrogenation reactions, the synthesis of methanol and higher alcohols from synthesis gas, etc. The copper-containing catalysts also may contain other metal oxides including chromium oxide, zinc oxide, titanium oxide, zirconium oxide, iron oxide, alumina, silica, etc., and mixtures of one or more of said oxides.

The hydrogenation of carboxylic acids and carboxylic esters to alcohols is known in the art, and various methods and catalysts have been suggested for effecting the hydrogenation reaction. For example, the ester may be reduced with lithium aluminum hydride or sodium and alcohol. A commonly practiced method involves the use of a copper-chromite-based hydrogenation catalyst. While copper chromite catalysts are commercially available and successful, the disposal of the spent copper chromite catalyst is a problem since chromium is present in the spent catalyst, and chromium is a highly toxic material which is subject to stringent EPA disposal regulations. Because of the stringent regulations, the cost of the manufacture, use and disposal of copper chromite catalysts has increased. It is anticipated that in the future, more stringent EPA regulations and escalating disposal costs will adversely affect the economics of using a copper-chromite-based catalyst.

U.S. Patent 2,091,800 describes a copper chromite/barium catalyst which is used in a process for hydrogenating esters at a temperature in the range of 200°C to 400°C by passing the acid and its esters over the hydrogenation catalyst. Other patents describing various types of copper chromite catalysts used in acid and ester hydrogenation processes include U.S. Patent Nos. 2,121,367; 2,782,243; 3,173,959; and 3,267,157.

U.S. Patent 3,894,054 describes the production of tetrahydrofuran by catalytic hydrogenation and dehydration of maleic anhydride using a catalyst composition which comprises a mixture obtained by calcining a silica-alumina catalyst and a copper-chromium-zinc catalyst. U.S. Patent 3,971,735 describes the preparation of methanol from syngas with a catalyst comprised of copper, zinc, aluminum and boron. The hydrogenation of esters to alcohols by contacting the ester with hydrogen and a catalyst comprising cobalt, zinc and copper under catalytic hydrogenation conditions is described in U.S. Patent 4,113,662. U.S. Patent 4,279,781 describes a methanol synthesis catalyst which comprises the oxides of copper and zinc and a minor amount of a thermal stabilizing metal oxide such as alumina. The copper to zinc metal-weight ratio is in the range of from 2:1 to 3.5:1. Catalysts comprising copper, cobalt, a metal selected from chromium, iron, vanadium or manganese, a rare earth metal and a small amount of an alkali or alkaline earth metal are described in U.S. Patent 4,291,126. Optionally, the catalyst may contain zinc and/or a noble metal and/or a binder selected from alumina, magnesia and cements. U.S. Patent 4,440,668 describes a three-component oxide catalyst based on copper, a metal from Group VIA, VIIA or VIIIA, and a metal of Group IVA or VA. The preferred catalyst is based on copper, cobalt and zirconium with the first two components being formed by co-precipitation in the presence of the oxide of the third component. Another multi-component catalytic system is described in U.S. Patent 4,513,100 which comprises zinc, chromium, copper, one or more alkaline metals and possibly one or more metals chosen from molybdenum, manganese, lanthanum, cerium, aluminum, titanium and vanadium.

U.S. Patent 4,535,071 describes a catalyst for methanol synthesis from syngas which comprises as catalytically active substances, copper oxide and zinc oxide and as a thermal stabilizing substance, aluminum oxide. Optimum yields of methanol are obtained when the atomic ratio of copper to zinc is between 2.8 and 3.8. Five-component catalyst compositions are described in U.S. Patent 4,551,444 and the essential components are copper, an iron group component, a component of elements 23-26, an alkaline metal compound and a precious metal compound. Catalysts comprising copper oxide and zinc oxide in a ratio of 8:1 to 1:1 are described in U.S. Patent 4,588,848 as being useful in synthesizing neoalcohols from neoacids. U.S. Patent 4,598,061 describes a catalyst for synthesis of methanol and alcohol mixtures from synthesis gas using a catalyst which contains, as an oxide precursor, copper oxide and zinc oxide; aluminum oxide as a thermal stabilizing substance; and at least one alkali metal compound. Catalysts comprising copper and cobalt, and optionally aluminum and/or zinc and/or sodium are utilized in U.S. Patent 4,675,343 for preparing primary aliphatic alcohols from hydrogen and carbon oxides. The catalysts contain a minimum of 3% cobalt. Catalysts containing the oxides of copper, zinc and alumina are described in U.S. Patent 4,704,480 as being useful in the production of aliphatic ketones and an optional consecutive production of the corresponding carbinols. More specifically, catalysts comprising the oxides of copper, zinc and alumina are utilized in Examples 1 and 11 of the patent and a catalyst comprising the oxides of copper and alumina is utilized in Example 12. Copper-zinc catalysts also are described in U.S. Patent 4,808,562, and the catalysts may contain alumina.

U.K. Patent 1,436,773 also describes copper oxide, zinc oxide catalysts obtained by coprecipitation which are suitable for use in the synthesis of methanol from synthesis gas. The ratio of copper to zinc in the catalyst is from 1:1 to 8:1, and the catalyst may contain a thermal stabilizer such as alumina. Japanese Patent 62-53740 apparently describes catalysts derived from the nitrates of copper, zinc, manganese/magnesium and aluminium.

German Offenlegungschrift 2,613,226 describes a continuous preparation of fatty alcohols by catalytic hydrogenation of relatively high molecular weight fatty acids and esters formed with low-molecular weight monohydric alcohols. The process utilizes hydrogen and a catalyst. The catalysts disclosed in the patent include copper chromite or copper-zinc-chromite and copper-zinc catalysts with or without known carrier substances.

Although many copper-containing catalysts have been described in the prior art, there continues to be a need for catalysts which are useful particularly in the hydrogenation of aldehydes, acids and esters, including diesters. It is also desirable to prepare catalysts useful in hydrogenation reactions which can be carried out in either a fixed bed or a fluidized bed reactor.

### Summary of the Invention

The present invention provides a process for preparing a hydrogenation catalyst comprising the oxides of copper, zinc and aluminium which comprises the steps of
(a) preparing a first aqueous solution containing at least one water-soluble copper salt and at least one water-soluble zinc salt;
(b) preparing a second solution containing at least one water-soluble basic aluminium salt and at least one alkaline precipitating agent;
(c) mixing the first and second solutions by simultaneously adding the two solutions to a vessel and by conducting the mixing of the two solutions at a pH above 7, the pH of the resulting mixture being controlled by adjusting the relative rates of addition of the two solutions, whereby an insoluble solid is formed;
(d) recovering the insoluble solid; and
(e) calcining the recovered solid,
wherein the atomic ratio of copper to zinc in the first aqueous solution is less than I, and wherein the catalyst recovered from the calcination of the removed solid in step (e) contains from 0.5 to 40% by weight of aluminium oxide.

### Description of the Drawings

Fig. 1 is a graph illustrating the methyl ester hydrogenation activity of the catalyst prepared in Examples 1-8 and 12 as well as control examples C-1 to C-5 and a commercial catalyst with respect to percent conversion.
Fig. 2 is a graph illustrating the relative methyl ester hydrogenolysis activity (with respect to saponification value) of the catalyst of Example 1 compared to the activity of several catalysts containing other metal combinations.

### Description of the Preferred Embodiments

In one embodiment, the present invention relates to the preparation of a catalyst in powder form comprising a major amount of the oxides of copper and zinc and a minor amount of aluminium oxide wherein the pore volume of pores of said powders having a diameter of greater than 80Å (i.e. 80 ångstroms or 8 nm) is at least 80% of the total pore volume. In one preferred embodiment, the pore volume of pores having a diameter of greater than 80Å (8 nm) is at least 85% of the total pore volume. In another embodiment, more than 75% of the pores have a diameter in excess of 120Å (12 nm).

All references to pore diameters and pore volumes in the specification and claims are based upon measurements utilizing mercury porosimetry. A typical method is described by R. Anderson, Experimental Methods in Catalytic Research, Academic Press, New York, 1968. The pore volumes are determined utilizing the powder forms of the catalysts in their oxide forms. That is, the pore diameters reported herein are obtained for the powder catalyst after calcination, but prior to any reduction of the oxide. Those skilled in the art often refer to the catalyst containing the metal oxides as the "oxide" or "oxidic precursor" form of the catalyst.

The powdered catalysts of the present invention which contain a major amount of the oxides of copper and zinc, and a minor amount of aluminium oxide, also may have an average surface area of at least 70 square meters per gram and more generally from 70 to 200 square meters per gram.

The powdered catalysts also may have an average particle diameter of from 8 to 28 microns (µm).

Preferably, the catalyst does not contain chromium and cobalt and, more preferably, also does not contain boron.

The amount of aluminium oxide contained in the catalyst of this invention is, as stated above, from 0.5 to 40% by weight; however, the catalyst will generally contain from 2% to 40%, e.g. from 3 to 40% by weight of aluminium oxide, and more often contains from 5% to 30% by weight of aluminium oxide. The catalyst of the invention also may have an atomic ratio of copper and zinc to aluminium of less than 15.

The catalyst prepared in accordance with the above method may have an atomic ratio of copper to zinc of at least 0.2. Such catalysts are prepared by maintaining the atomic ratio of copper to zinc in the first aqueous solution within the indicated range. In another embodiment, the atomic ratio of copper to zinc in the first aqueous solution is 0.85 or less. More preferably, the atomic ratio of copper to zinc is from 0.25 to 0.85. The amount of alumina contained in the catalyst recovered from the calcination of the removed solid in step (e) may contain from 2% to 40% by weight of aluminium oxide, and more generally, from 5% to 30% by weight of aluminium oxide.

A mixture of the first and second solutions described above is obtained by simultaneously mixing the two solutions, by simultaneously adding the two solutions to a vessel. The mixing of the first and second solutions in step (e) is conducted at a pH above 7.0. The pH of the resulting mixture can be controlled by varying the rate of addition of the second solution which contains an alkaline material. As the rate of addition of the second solution increases, the pH of the resulting mixture increases.

The water-soluble copper and zinc salts utilized to form the first solution are copper and zinc salts such as the nitrates, acetates, sulfates, chlorides, etc. It is presently preferred, however, to use the nitrates of copper and zinc in the formation of the first solution. Any water-soluble aluminium salt can be utilized to prepare the second solution, and the aluminium salt generally is a basic aluminium salt such as sodium aluminate.

The second solution also contains at least one alkaline material which may be a soluble base such as sodium hydroxide, sodium carbonate, ammonium hydroxide, ammonium carbonate, etc., and mixtures thereof. The amount of alkaline material included in the second solution may be varied over a wide range, and the amount of alkaline materials should be sufficient to provide an alkaline solution which, when added to the first solution will result in a mixture having the desired pH. The pH of the mixture obtained by mixing the first and second solutions should preferably be within the range of from 7.0 to 9.0 and more preferably is at least 7.5. As noted above, the pH of the mixture can be maintained as desired by adjusting the relative addition rates of the two solutions. Additionally, the mixture obtained from the first and second solutions should preferably be maintained at a temperature of from 50-80°C. A precipitate is formed and recovered by techniques well known in the art such as by filtration, centrifugation, etc. The recovered precipitate preferably is washed with water to remove impurities, dried by heating to a temperature of up to 150°C, and finally calcined. The recovered precipitate is calcined at a temperature in the range of from 475°C to 700°C for a period of 30 to 120 minutes. Generally, calcination at a temperature of 500°C for 30 minutes is sufficient.

The following examples 1-8 illustrate the above-described process for preparing the hydrogenation catalyst of the present invention. Unless otherwise indicated in the examples and elsewhere in the specification and claims, all parts and percentages are by weight, temperatures are in degrees Centigrade, and pressures are at or near atmospheric. Examples 9-12 are comparative examples.

### Example 1

A first solution is prepared from 710 parts of an aqueous copper nitrate solution containing 16.3% copper, 620 parts of zinc nitrate tetrahydrate and 1600 parts water. A second solution is prepared by dissolving 330 parts sodium aluminate (23% Al) and 350 parts soda ash in 2500 parts water. The two solutions are mixed in a reaction vessel that contains 5000 parts water at 70°C. The reaction temperature is maintained at 70°C (±5°C) and the pH is maintained at about 7.5. A precipitate is formed and recovered by filtration. The recovered precipitate is washed with water, dried at 125°C and finally calcined at 500°C (±10°C) for 30 minutes. The physical and chemical characteristics of the calcined product are given in Table I.

### Example 2

A first solution is prepared from 467 parts of an aqueous copper nitrate solution containing 16.3% copper, 933 parts of an aqueous zinc nitrate solution containing 16.5% zinc and 200 parts water. A second solution is prepared by dissolving 35 parts sodium aluminate (23% Al), 126 parts of an aqueous solution of sodium hydroxide (50% NaOH) and 413 parts soda ash in 1200 parts water. The two solutions are mixed in a reaction vessel that contains 5000 parts water at 70°C. The reaction temperature is maintained at 70°C (±5°C), and the pH is maintained at about 7.5. A precipitate is formed and recovered by filtration. It is then washed with water, dried at 125°C and finally calcined at 500°C (±10°C) for 30 minutes. The physical and chemical characteristics of the calcined product are given in Table I.

### Example 3

A first solution is prepared from 351 parts of an aqueous copper nitrate solution containing 16.3% copper, 1050 parts of an aqueous zinc nitrate solution containing 16.5% zinc and 220 parts water. A second solution is prepared by dissolving 35 parts sodium aluminate (23% Al), 126 parts of an aqueous solution of sodium hydroxide (50% NaOH) and 413 parts soda ash in 1200 parts water. The two solutions are mixed in a reaction vessel that contains 5000 parts water at 70°C. The reaction temperature is maintained at 70°C (±5°C), and the pH is maintained at about 7.5. A precipitate is formed and recovered by filtration. It is then washed with water, dried at 125°C and calcined at 500°C for 30 minutes. The physical and chemical characteristics of the calcined product are given in Table I.

### Example 4

A first solution is prepared from 622 parts of an aqueous copper nitrate solution containing 16.3% copper, 778 parts of an aqueous zinc nitrate solution containing 16.5% zinc and 200 parts water. A second solution is prepared by dissolving 35 parts sodium aluminate (23% Al), 86 parts of an aqueous solution of sodium hydroxide (50% NaOH) and 413 parts soda ash in 1237 parts water. The two solutions are mixed in a reaction vessel that contains 5000 parts water at 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table I.

### Example 5

A first solution is prepared from 350 parts of an aqueous copper nitrate solution containing 16.3% copper, 1050 parts of an aqueous zinc nitrate solution containing 16.5% zinc and 200 parts water. A second solution is prepared by dissolving 330 parts sodium aluminate (23% Al), 21 parts of an aqueous solution of sodium hydroxide (50% NaOH) and 320 parts soda ash in 1601 parts water. The two solutions are mixed in a reaction vessel that contains 5000 parts water at 70°C. The reaction temperature is maintained at 70°C, and the pH is maintained at about 7.5. A precipitate is formed and recovered by filtration. It is then washed with water, dried, at 125°C and calcined at 500°C for 30 minutes. The physical and chemical characteristics of the calcined product are given in Table I.

### Example 6

A first solution is prepared from 710 parts of an aqueous copper nitrate solution containing 16.5% zinc and 1452 aqueous zinc nitrate solution containing 16.5% zinc and 1452 parts water. A second solution is prepared by dissolving 330 parts sodium aluminate (23% Al), 30 parts of an aqueous solution of sodium hydroxide (50% NaOH) and 350 parts soda ash in 2252 parts water. The two solutions are mixed in a reaction vessel that contains 5000 parts water at 70°C. The reaction temperature is maintained at 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table I.

### Example 7

A first solution is prepared from 324 parts of an aqueous copper nitrate solution containing 16.3% copper, 1291 parts of an aqueous zinc nitrate solution containing 16.5% zinc and 1489 parts water. A second solution is prepared by dissolving 330 parts sodium aluminate (23% Al), 10 parts of an aqueous solution of sodium hydroxide (50% NaOH) and 350 parts soda ash in 2212 parts water. The two solutions are mixed in a reaction vessel that contains 5000 parts water at 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table IA.

### Example 8

A first solution is prepared from 710 parts of an aqueous copper nitrate solution containing 16.3% copper, 899 parts of an aqueous zinc nitrate solution containing 16.5% zinc and 1414 parts water. A second solution is prepared by mixing 286 parts of aqueous solution of sodium aluminate (13% Al), 21 parts of an aqueous solution of sodium hydroxide (50% NaOH) and 350 parts soda ash in 1100 parts water. The two solutions are mixed in a reaction vessel that contains 5000 parts water at 70°C. The reaction temperature is maintained at 70°C, and the pH is maintained at about 7.5. A precipitate is formed and recovered by filtration. It is then washed with water, dried at 125°C and calcined at 600°C for 45 minutes. The physical and chemical characteristics of the calcined product are given in Table IA.

### Example 9 (Comparative)

A first solution is prepared from 1000 parts of an aqueous nitrate solution containing 16.3% copper, 335 parts of an aqeuous zinc nitrate solution containing 16.5% zinc and 1600 parts water. A second solution is prepared by dissolving 113 parts sodium aluminate (23% Al) and 490 parts soda ash in 2500 parts water. The two solutions are mixed in a reaction vessel that contains 5000 parts water at 70°C. The reaction temperature is maintained at 70°C (±5°C), and the pH of the mixture is maintained at 7.5. A precipitate is formed and recovered by filtration. It is then washed with water, dried at 125°C and finally calcined at 500°C (±10°C) for 75 minutes. The physical and chemical characteristics of the calcined product are shown in Table IA.

### Example 10 (Comparative)

A first solution is prepared from 1000 parts of an aqueous copper nitrate solution containing 16.4% copper, 253 parts of an aqueous zinc nitrate solution containing 16.5% zinc and 1600 parts water. A second solution is prepared by dissolving 37 parts sodium aluminate (23% Al) and 450 parts soda ash in 2300 parts water. The two solutions are mixed in a reaction vessel that contains 5000 parts water at 70°C. The reaction temperature is maintained at 70°C (±5°C), and the pH of the mixture is maintained at about 7.5. A precipitate is formed and recovered by filtration. It is then washed, with water, dried at 125°C and finally calcined at 480°C (±10°C) for 50 minutes. The physical and chemical characteristics of the calcined product are shown in Table IA.

### Example 11 (Comparative)

A first solution is prepared from 1000 parts of an aqueous copper nitrate solution containing 16.3% copper, 200 parts of an aqueous zinc nitrate solution containing 16.5% zinc and 200 parts water. A second solution is prepared by dissolving 35 parts sodium aluminate (23% Al), 126 parts of an aqueous sodium hydroxide solution (50% NaOH), and 413 parts soda ash in 1000 parts water. The two solutions are mixed in a reaction vessel that contains 5000 parts water at 70°C. The reaction temperature is maintained at 70°C (±5°C), and the pH of the mixture is maintained at about 7.5. A precipitate is formed and recovered by filtration. It is then washed with water, dried at 125°C and finally calcined at 500°C (±10°C) for 30 minutes. The physical and chemical characteristics of the calcined product are shown in Table IA.

### Example 12 (Comparative)

A first solution is prepared from 701 parts of an aqueous copper nitrate solution containing 16.3% copper, 702 parts of an aqueous zinc nitrate solution containing 16.5% zinc and 208 parts water. A second solution is prepared by dissolving 415 parts soda ash in 1000 parts water. The two solutions are mixed in a reaction vessel that contains 5000 parts water in which 100 parts of a hydrated alumina powder (Kaiser Versal® 850) is dispersed. The reaction temperature is maintained at 70°C (±5°C), and the pH is maintained at about 7.5. A precipitate is formed and recovered by filtration. It is then washed with water, dried at 125°C and calcined at 500°C (±10°C) for 30 minutes. The physical and chemical characteristics of the calcined product are given in Table IA.

**TABLE I**

| | Example | | | | | |
|---|---|---|---|---|---|---|
| | 1 | 2 | 3 | 4 | 5 | 6 |
| Cu/Zn Molar Ratio | 0.82 | 0.52 | 0.36 | 0.84 | 0.35 | 0.81 |
| (Cu+Zn)/Al Ratio | 1.22 | 12.3 | 8.79 | 11.89 | 1.46 | 2.06 |
| Surf. Area (M²/g) | 173 | 90.9 | 89.1 | 85.3 | 165 | 143 |
| He Density (g/cc) | 4.32 | 5.16 | 4.62 | 4.77 | 3.73 | 3.96 |
| Hg Density@18.5 PSI φ | 0.44 | 0.50 | 0.50 | 0.49 | 0.57 | 0.49 |

| Pore Diameter (A°)* | | | | | | |
|---|---|---|---|---|---|---|
| Up to 60 | 10.2 | 3.4 | 5.2 | 0 | 6.2 | 6.8 |
| Up to 80 | 11.4 | 4.4 | 6 | 0 | 9.5 | 7.7 |
| Up to 100 | 12.7 | 5.6 | 7.6 | 0 | 12.9 | 8.9 |
| Up to 120 | 13.5 | 7.3 | 9.6 | 0 | 15.2 | 10.1 |
| Up to 350 | 15.6 | 19.4 | 20.9 | 18.7 | 20.6 | 18.1 |
| Up to 700 | 16.9 | 23.7 | 27.5 | 25.5 | 22.7 | 24.5 |
| Up to 1000 | 17.9 | 25.4 | 30.2 | 28 | 24.1 | 27.5 |
| Up to 10000 | 48.3 | 43.1 | 48.6 | 45.8 | 42.2 | 47.1 |

| Average Particle | | | | | | |
|---|---|---|---|---|---|---|
| Size (µm) | 22 | 15.8 | 15.2 | 15.1 | 13.4 | 18.7 |

| Chemical Analysis at 500°C (% wt.) | | | | | | |
|---|---|---|---|---|---|---|
| Copper | 23.8 | 24.8 | 18.2 | 33.3 | 13.6 | 22.93 |
| Zinc | 30.0 | 48.8 | 51.5 | 40.6 | 39.6 | 29.00 |
| Aluminum | 14.6 | 2.50 | 3.30 | 2.60 | 15.2 | 14.40 |

| | | | | | | |
|---|---|---|---|---|---|---|
| * Cumulative percentage. 1 Å = 0.1 nm | | | | | | |
| φ 18. 5 PSI = 127.6 kPa | | | | | | |

**TABLE IA**

| | Example | | | | | |
|---|---|---|---|---|---|---|
| | 7 | 8 | 9 | 10 | 11 | 12 |
| Cu/Zn Molar Ratio | 0.26 | 0.82 | 1.94 | 4.08 | 5.16 | 1.07 |
| (Cu+Zn)/Al Ratio | 1.75 | 2.29 | 2.07 | 10.4 | 12.3 | 2.33 |
| Surf. Area (M²/g) | 179 | ---- | 79.9 | 70.1 | 69.0 | 48.9 |
| He Density (g/cc) | 4.37 | 4.39 | 4.92 | 5.10 | 5.20 | 4.00 |
| Hg Density@18.5 PSI φ | 0.47 | 0.41 | 0.51 | 0.65 | 0.78 | 1.17 |

| Pore Diameter (A°)* | | | | | | |
|---|---|---|---|---|---|---|
| Up to 60 | 2.8 | 6.8 | 17 | 5 | 3.4 | 13.3 |
| Up to 80 | 8.6 | 8.7 | 17 | 5.2 | 4.2 | 14.8 |
| Up to 100 | 15.6 | 9.8 | 17 | 6.3 | 4.9 | 16.3 |
| Up to 120 | 20.2 | 10.5 | 17.3 | 8.3 | 6 | 17.9 |
| Up to 350 | 32.2 | 14.3 | 25.3 | 26.1 | 31.7 | 52 |
| Up to 700 | 35.3 | 17.4 | 33.7 | 34.8 | 48.7 | 71.8 |
| Up to 1000 | 36.2 | 19.3 | 40.3 | 39.2 | 55.7 | 75.7 |
| Up to 10000 | 43.5 | 36 | 72.9 | 49.5 | 84.1 | 83.8 |

| Average Particle | | | | | | |
|---|---|---|---|---|---|---|
| Size (µm) | 17.1 | 17.4 | 23 | 22.3 | 16.9 | 57.5 |

| Chemical Analysis at 500°C (% wt.) | | | | | | |
|---|---|---|---|---|---|---|
| Copper | 10.96 | 25.6 | 44.5 | 58.7 | 61.9 | 28.1 |
| Zinc | 43.90 | 32.0 | 23.6 | 14.8 | 12.2 | 27.0 |
| Aluminum | 13.05 | 7.1 | 2.96 | 2.6 | 15.2 | 9.9 |

| | | | | | | |
|---|---|---|---|---|---|---|
| * Cumulative percentage. 1Å = 0.1 nm | | | | | | |
| φ 18.5 PSI = 127.6 kPa. | | | | | | |

The following examples C-1 to C-6 illustrate the preparation of control catalysts which are not within the present invention but are presented herein for comparison purposes.

### Example C-1

### (Cu/Zn)

A first solution is prepared from 1000 parts of an aqueous copper nitrate solution containing 16.3% copper, 1017 parts of an aqueous zinc nitrate solution containing 16.5% zinc and 689 parts water. A second solution is prepared from 374 parts of soda ash in 1012 parts water. The two solutions are mixed in a reaction vessel that contains 2000 parts water at 45°C. The pH is maintained at about 7.5, and a precipitate is formed and recovered by filtration. It is then washed with water, dried at 125°C and calcined at 500°C for 30 minutes. The physical and chemical characteristics of the calcined product are given in Table IB.

### Example C-2

### (Cu/Al)

A first solution is prepared from 1010 parts of an aqueous copper nitrate solution containing 16.3% copper and 1640 parts water. A second solution is prepared from 374 parts of soda ash in 1012 parts water. The two solutions are mixed in a reaction vessel that contains 2000 parts water at 45°C. The pH is maintained at about 7.5, and a precipitate is formed and recovered by filtration. It is then washed with water, dried at 125°C and calcined at 500°C for 30 minutes. The physical and chemical characteristics of the calcined product are given in Table IB.

### Example C-3

### (Zn/Al)

A first solution is prepared from 1015 parts of an aqueous zinc nitrate solution containing 16.5% zinc and 1745 parts water. A second solution is prepared by dissolving 342 parts sodium aluminate (23% Al) and 250 parts soda ash in 2210 parts water. The two solutions are mixed in a reaction vessel that contains 5000 parts water at 70°C. The reaction temperature is maintained at 70°C and the pH is maintained at about 7.5. A precipitate is formed and recovered by filtration. It is then washed with water, dried at 125°C and calcined at 500°C for 30 minutes. The physical and chemical characteristics of the calcined product are given in Table IB.

### Example C-4

### (Cu/Zn/Al/Co)

A first solution is prepared from 500 parts of an aqueous copper nitrate solution containing 16.3% copper, 254 parts of an aqueous zinc nitrate solution containing 16.5% zinc, and 17 parts of cobalt nitrate hexahydrate. A second solution is prepared by mixing 115 parts of sodium aluminate (23% Al) and 250 parts soda ash in 2485 parts water. The two solutions are mixed in a reaction vessel that contains 2140 parts water at 70°C. The reaction temperature is maintained at about 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table IB.

### Example C-5

### (Cu/Zn/Co)

A first solution is prepared from 501 parts of an aqueous copper nitrate solution containing 16.3% copper, 255 parts of an aqueous zinc nitrate solution containing 16.5% zinc, and 34 parts of cobalt nitrate hexahydrate. A second solution is prepared from 365 parts soda ash in 2487 parts water. The two solutions are mixed in a reaction vessel that contains 2140 parts water at 70°C. The reaction temperature is maintained at about 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table IB.

### Example C-6

### (Cu/Cr/Mn)

A first solution made up of 600 parts of a copper nitrate solution (16.3% Cu), 31.5 parts of a manganese nitrate solution (15.5% Mn) and 175 parts chromic acid (CrO₃) and a second solution made up of 362 parts concentrated ammonium hydroxide and 132 parts water are added to a reaction vessel, containing 1500 parts water at 50°C, to form a precipitate. The precipitate is collected by filtration and washed with water. After drying at 150°C for 12 hours, the material is calcined at 420°C for 30 minutes. The physical and chemical characteristics of the calcined product are given in Table IB.

**TABLE IB**

| | Example | | | | | |
|---|---|---|---|---|---|---|
| | C-1 | C-2 | C-3 | C-4 | C-5 | C-6 |
| Cu/Zn Molar Ratio | 1.01 | 1011 | 0.00 | 2.11 | 2.14 | ---- |
| (Cu+Zn)/Al Ratio | ---- | 1.25 | 0.69 | 2.28 | ---- | ---- |
| Surf. Area (M²/g) | 16.6 | 82.4 | 158 | 98.3 | ---- | 60 |
| He Density (g/cc) | 5.06 | 3.56 | 4.09 | 4.01 | 5.81 | 4.9 |
| Hg Density@18.5 PSIφ | 1.08 | 0.47 | 0.46 | 0.38 | 1.21 | 1.6 |

| Pore Diameter (A°)* | | | | | | |
|---|---|---|---|---|---|---|
| Up to 60 | 6.2 | 0 | 1.1 | 2 | 7.6 | |
| Up to 80 | 6.2 | 0.3 | 3.3 7 | 2 | 7.6 | |
| Up to 100 | 6.2 | 0.9 | 7.4 | 2 | 7.6 | |
| Up to 120 | 6.2 | 1.6 | 10.8 | 2 | 7.6 | |
| Up to 350 | 6.9 | 13.6 | 20.2 | 7 | 29.9 | |
| Up to 700 | 32.2 | 25.1 | 23.8 | 14.6 | 43.5 | |
| Up to 1000 | 42.5 | 32 | 25.7 | 19.1 | 48.3 | |
| Up to 10000 | 75.4 | 87.1 | 54.3 | 53.2 | 76.5 | |

| Average Particle | | | | | | |
|---|---|---|---|---|---|---|
| Size (µm) | 19.5 | 17.2 | 18.3 | 22.3 | 12.6 | 22 |

| Chemical Analysis at 500°C (% wt.) | | | | | | |
|---|---|---|---|---|---|---|
| Copper | 34.5 | 49.1 | 0.0 | 39.2 | 51.0 | 35.7 |
| Zinc | 35.0 | 0.05 | 29.4 | 19.1 | 24.6 | Cr=31.7 |
| Aluminum | ---- | 16.7 | 17.6 | 11.4 | ---- | ---- |
| Other | ---- | ---- | ---- | Co=3.1 | Co=4.2 | Mn=3.7 |

| | | | | | | |
|---|---|---|---|---|---|---|
| * Cumulative percentage. 1Å = 0.1 nm | | | | | | |
| φ 18.5 PSI = 127-6 kPa | | | | | | |

The catalysts of the present invention which contain copper, zinc and alumina have been found to be particularly useful for hydrogenating aldehydes, ketones, carboxylic acids and carboxylic esters to alcohols. Generally, these catalysts do not contain chromium or cobalt. In one embodiment, the atomic ratio of copper to zinc in the catalysts is at least 0.5, and in one preferred embodiment, the ratio is less than 0.85, and the catalyst is also free of boron. In another preferred embodiment, the hydrogenation catalyst is characterized by an atomic ratio of copper to zinc of less than 0.85, the catalyst is free of chromium, cobalt and boron, and the catalyst is prepared by the preferred process as illustrated in Examples 1-8.

The catalysts of the present invention which are prepared as powders may be utilized in slurry-(liquid-) phase hydrogenation processes. Alternatively, the powders can be processed into shapes such as pellets and used in fixed bed reactors. In one embodiment, carboxylic acids and carboxylic esters can be converted to alcohols in excellent yields. A wide variety of acids, particularly esters of carboxylic acids can be treated with the catalyst of the present invention to produce alcohols. The esters may be monoesters or diesters. Among the acids which may be hydrogenated to the corresponding alcohols without isolating the ester include stearic acids and caproic acids. Esters derived from the alcohols of higher molecular weight carboxylic acids are hydrogenated more rapidly and at lower temperatures than the esters derived from the lighter alcohols. Examples of esters which may be hydrogenated with the catalyst of the present invention include the methyl ester of coconut fatty acid, methyl stearate, methyl oleate, ethyl laurate, ethyl myristate, the diethyl ester of ethyl malonic acid, diethyl succinate, di-n-butyl glutarate, diethyl sebacate. As noted, the esters are converted to alcohols, and examples of such conversions include: ethyl laurate to lauryl alcohol; ethyl myristate to myristyl alcohol; ethyl valerate to n-amyl alcohol; methyl caproate to n-hexyl alcohol, etc.

Examples of aldehydes which may be hydrogenated with the catalyst of the present invention include: butyraldehyde, furfural, 2-ethyl-hexanal, dodecanal, tetradecanal, etc. Examples of ketones include acetone, acetophenone, etc.

The hydrogenation reactions which are conducted in the presence of the catalyst of the present invention are carried out at temperatures of from 250°C to 350°C and at pressures of from 1500 psi to 4500 psi (10 342 to 31 026 kPa)

In one preferred embodiment, the hydrogenation reaction is conducted in a batch or continuous ebullated bed reactor. In this process, the catalyst powder particles are slurried with the aldehyde, ketone, carboxylic acid or carboxylic ester to be reduced, and there is intimate contact between the catalyst and the liquid. When one of the preferred catalysts of the present invention wherein the atomic ratio of copper to zinc is less than 0.85, and the catalyst is prepared by the preferred coprecipitation procedure such as illustrated in Examples 1-8, is used in a batch ebullated reactor, high yields of alcohols are obtained in shorter times, and the slurry, upon completion of the hydrogenation reaction is easily filtered.

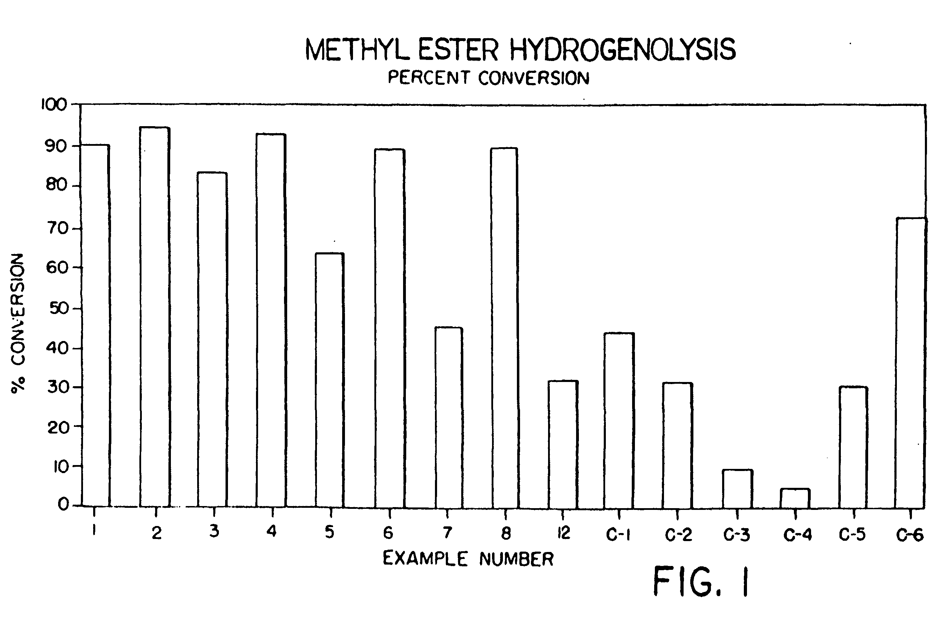

The effectiveness of the catalyst of Examples 1-8 of the present invention as hydrogenation catalysts is illustrated by utilizing the catalysts to hydrogenate coconut methyl-ester at 280°C, 3000 psig (20 684 kPa gauge) hydrogen and 1.8 weight percent catalyst loading, and measuring the percent conversion to the alcohol after the hydrogenation reaction has been conducted for 60 minutes. The results are summarized in the graph of Fig. 1. Also contained in Fig. 1 are the results of the hydrogenation reaction conducted with other catalysts which are not within the scope of the present invention which have been identified above as Examples 12 and C-1 to C-6.

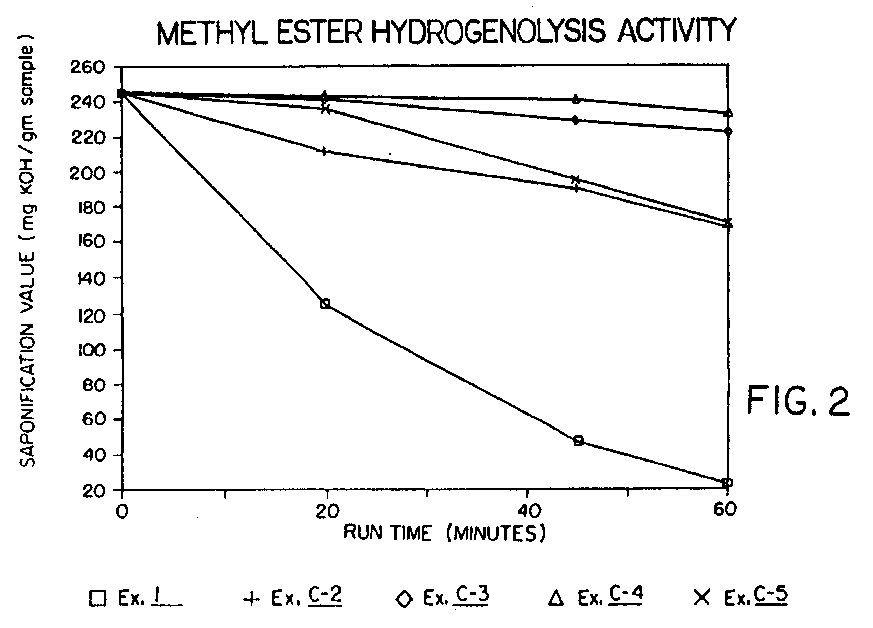

The hydrogenation activity of the catalyst of Example 1 also has been compared to the catalysts of Examples C-2 through C-5 by carrying out hydrogenation reactions of coconut methyl ester at 280°C, 3000 psig (20 684 kPa gauge) of hydrogen and 1.8 weight percent catalyst loading. The effectiveness of the catalyst is determined by measuring the saponification value for each of the samples pursuant to A.O.C.S. official method Cd 3-25, revised April, 1966, reapproved 1973. Under these specific test conditions, saponification values of below about 50 indicate good hydrogenation activity. The results of this test are shown in Fig. 2, and the results demonstrate the improved hydrogenation activity of the catalyst of Example 1 when compared to the catalysts of Examples C-2 to C-5.

While the invention has been explained in relation to its preferred embodiments, it is to be understood that various modifications thereof will become apparent to those skilled in the art upon reading the specification. Therefore, it is to be understood that the invention disclosed herein is intended to cover such modifications.

## Claims

1. A process for preparing a hydrogenation catalyst comprising the oxides of copper, zinc and aluminium which comprises the steps of
(a) preparing a first aqueous solution containing at least one water-soluble copper salt and at least one water-soluble zinc salt;
(b) preparing a second solution containing at least one water-soluble basic aluminium salt and at least one alkaline precipitating agent;
(c) mixing the first and second solutions by simultaneously adding the two solutions to a vessel and by conducting the mixing of the two solutions at a pH above 7, the pH of the resulting mixture being controlled by adjusting the relative rates of addition of the two solutions, whereby an insoluble solid is formed;
(d) recovering the insoluble solid; and
(e) calcining the recovered solid,
wherein the atomic ratio of copper to zinc in the first aqueous solution is less than 1, and wherein the catalyst recovered from the calcination of the removed solid in step (e) contains from 0.5 to 40% by weight of aluminium oxide.

2. The process of claim 1 wherein the atomic ratio of copper to zinc in the first aqueous solution is at least 0.2.

3. The process of claim 1 or 2 wherein the atomic ratio of copper to zinc in the first aqueous solution is less than 0.85.

4. The process of claim 1 wherein the atomic ratio of copper to zinc in the first aqueous solution is from 0.25 to 0.85.

5. The process of claim 1 wherein the catalyst recovered from the calcination of the removed solid in step (e) contains from 3 to 40% by weight of aluminium oxide.

6. The process of claim 5 wherein the catalyst recovered from the calcination of the removed solid in step (e) contains from 5 to 30% by weight of aluminium oxide.

7. The process of any of claims 1 to 6 wherein the amount of aluminium salt in the second solution is sufficient to produce a catalyst wherein the atomic ratio of copper and zinc to aluminium is less than 15.

8. The process of any of claims 1 to 7, wherein the water-soluble salt of copper is copper nitrate.

9. The process of any of claims 1 to 8, wherein the water-soluble salt of zinc is zinc nitrate.

10. The process of any of claims 1 to 9, wherein the water-soluble aluminium salt is sodium aluminate.

11. The process of any of claims 1 to 10, wherein the alkaline precipitating agent is sodium hydroxide, sodium carbonate, ammonium hydroxide or ammonium carbonate.

12. The process of claim 3 or 4, wherein the resultant catalyst is free of chromium and cobalt.

13. The process of claim 12, wherein the resultant catalyst is also free of boron.

14. The process of any of claims 1 to 13, wherein the mixture obtained in step (c) is maintained at a temperature of from 50°C to 80°C.

15. The process of any of claims I to 14, wherein the pH of the mixture obtained in step (c) is maintained at about 7.5.

16. The process of any of claims I to 15, wherein the solid recovered in step (d) is washed with water and dried at a temperature of up to 150°C before being calcined.

17. The process of any of claims 1 to 16, wherein step (e) is effected by heating the solid at a temperature of from 475°C to 700°C for a period of from 30 to 120 minutes.

## Patentansprüche

1. Verfahren zur Herstellung eines Hydrierkatalysators umfassend die Oxide von Kupfer, Zink und Aluminium, umfassend die folgenden Schritte:
a) Herstellen einer ersten wässrigen Lösung enthaltend wenigstens ein wasserlösliches Kupfersalz und wenigstens ein wasserlösliches Zinksalz;
b) Herstellen einer zweiten Lösung enthaltend wenigstens ein wasserlösliches basisches Aluminiumsalz und wenigstens ein alkalisches Ausfällungsmittel;
c) Mischen der ersten und zweiten Lösung durch gleichzeitiges Zugeben der zwei Lösungen zu einem Behälter und Durchführen der Mischung der zwei Lösungen bei einem pH-Wert oberhalb von 7, wobei der pH-Wert der resultierenden Mischung gesteuert wird, indem die relative Geschwindigkeit der Zugabe der zwei Lösungen eingestellt wird, wodurch ein unlöslicher Feststoff gebildet wird;
d) Rückgewinnen des unlöslichen Feststoffes; und
e) Kalzinieren des rückgewonnenen Festkörpers,
wobei das Atomverhältnis von Kupfer zu Zink in der ersten wässrigen Lösung weniger als 1 beträgt, und wobei der aus der Kalzinierung des entfernten Festkörpers in Schritt (e) zurückgewonnene Katalysator zwischen 0,5 bis 40 Gew-% Aluminiumoxid enthält.

2. Verfahren nach Anspruch 1, wobei das Atomverhältnis von Kupfer zu Zink in der ersten wässrigen Lösung wenigstens 0,2 beträgt.

3. Verfahren nach Anspruch 1 oder 2, wobei das Atomverhältnis von Kupfer zu Zink in der ersten wässrigen Lösung weniger als 0,85 beträgt.

4. Verfahren nach Anspruch 1, wobei das Atomverhältnis von Kupfer zu Zink in der ersten wässrigen Lösung zwischen 0,25 bis 0,85 beträgt.

5. Verfahren nach Anspruch 1, wobei der aus der Kalzinierung des entfernten Festkörpers in Schritt (e) zurückgewonnene Katalysator zwischen 3 bis 40 Gew-% Aluminiumoxid enthält.

6. Verfahren nach Anspruch 5, wobei der aus der Kalzinierung des entfernten Festkörpers in Schritt (e) zurückgewonnene Katalysator zwischen 5 bis 30 Gew-% Aluminiumoxid enthält.

7. Verfahren nach einem der Ansprüche 1 bis 6, wobei die Menge Aluminiumsalz in der zweiten Lösung ausreichend ist, um einen Katalysator zu erzeugen, wobei das Atomverhältnis von Kupfer und Zink zu Aluminium weniger als 15 beträgt.

8. Verfahren nach einem der Ansprüche 1 bis 7, wobei das wasserlösliche Salz von Kupfer Kupfernitrat ist.

9. Verfahren nach einem der Ansprüche 1 bis 8, wobei das wasserlösliche Salz von Zink Zinknitrat ist.

10. Verfahren nach einem der Ansprüche 1 bis 9, wobei das wasserlösliche Aluminiumsalz Natriumaluminat ist.

11. Verfahren nach einem der Ansprüche 1 bis 10, wobei das alkalische Ausfällungsmittel Natriumhydroxid, Natriumkarbonat, Ammoniumhydroxid oder Ammoniumkarbonat ist.

12. Verfahren nach Anspruch 3 oder 4, wobei der resultierende Katalysator kein Chrom und Kobalt aufweist.

13. Verfahren nach Anspruch 12, wobei der resultierende Katalysator auch kein Bor aufweist.

14. Verfahren nach einem der Ansprüche 1 bis 13, wobei die in Schritt (c) erhaltene Mischung bei einer Temperatur von 50 °C bis 80 °C gehalten wird.

15. Verfahren nach einem der Ansprüche 1 bis 14, wobei der pH-Wert der Mischung, welche in Schritt (d) erhalten wird, auf ungefähr 7,5 gehalten wird.

16. Verfahren nach einem der Ansprüche 1 bis 15, wobei der in Schritt (b) zurückgewonnene Festkörper mit Wasser gewaschen wird und bei einer Temperatur von bis zu 150 °C getrocknet wird, bevor er kalziniert wird.

17. Verfahren nach einem der Ansprüche 1 bis 16, wobei Schritt (e) durch Erwärmen des Festkörpers bei einer Temperatur von zwischen 475 °C bis 700 °C für einen Zeitraum von 30 bis 120 Minuten erwärmt wird.

## Revendications

1. Procédé de préparation d'un catalyseur d'hydrogénation comprenant les oxydes de cuivre, de zinc et d'aluminium qui comprend les étapes consistant à :
(a) préparer une première solution aqueuse contenant au moins un sel de cuivre soluble dans l'eau et au moins un sel de zinc soluble dans l'eau ;
(b) préparer une seconde solution contenant au moins un sel d'aluminium basique soluble dans l'eau et au moins un agent de précipitation alcalin ;
(c) mélanger la première et la seconde solutions en ajoutant simultanément les deux solutions dans un récipient et en réalisant le mélange des deux solutions à un pH supérieur à 7, le pH du mélange obtenu étant contrôlé par le réglage des taux d'addition relatifs des deux solutions, moyennant quoi un solide insoluble est formé ;
(d) récupérer le solide insoluble ; et
(e) calciner le solide récupéré,
dans lequel le rapport atomique du cuivre par rapport au zinc dans la première solution aqueuse est inférieur à 1, et dans lequel le catalyseur récupéré à partir de la calcination du solide éliminé à l'étape (e) contient de 0,5 à 40 % en poids d'oxyde d'aluminium.

2. Procédé selon la revendication 1 dans lequel le rapport atomique du cuivre par rapport au zinc dans la première solution aqueuse est d'au moins 0,2.

3. Procédé selon la revendication 1 ou 2 dans lequel le rapport atomique du cuivre par rapport au zinc dans la première solution aqueuse est inférieur à 0,85.

4. Procédé selon la revendication 1 dans lequel le rapport atomique du cuivre par rapport au zinc dans la première solution aqueuse est compris entre 0,25 et 0,85.

5. Procédé selon la revendication 1 dans lequel le catalyseur récupéré à partir de la calcination du solide éliminé à l'étape (e) contient de 3 à 40 % en poids d'oxyde d'aluminium.

6. Procédé selon la revendication 5 dans lequel le catalyseur récupéré à partir de la calcination du solide éliminé à l'étape (e) contient entre 5 et 30 % en poids d'oxyde d'aluminium.

7. Procédé selon l'une quelconque des revendications 1 à 6 dans lequel la quantité de sel d'aluminium dans la seconde solution est suffisante pour produire un catalyseur dans lequel le rapport atomique du cuivre et du zinc par rapport à l'aluminium est inférieur à 15.

8. Procédé selon l'une quelconque des revendications 1 à 7 dans lequel le sel de cuivre soluble dans l'eau est le nitrate de cuivre.

9. Procédé selon l'une quelconque des revendications 1 à 8 dans lequel le sel de zinc soluble dans l'eau est le nitrate de zinc.

10. Procédé selon l'une quelconque des revendications 1 à 9 dans lequel le sel d'aluminium soluble dans l'eau est l'aluminate de sodium.

11. Procédé selon l'une quelconque des revendications 1 à 10 dans lequel l'agent de précipitation alcalin est l'hydroxyde de sodium, le carbonate de sodium, l'hydroxyde d'ammonium ou le carbonate d'ammonium.

12. Procédé selon la revendication 3 ou 4 dans lequel le catalyseur obtenu ne contient pas de chrome et de cobalt.

13. Procédé selon la revendication 12 dans lequel le catalyseur obtenu ne contient également pas de bore.

14. Procédé selon l'une quelconque des revendications 1 à 13 dans lequel le mélange obtenu à l'étape (c) est maintenu à une température allant de 50°C à 80 C.

15. Procédé selon l'une quelconque des revendications 1 à 14 dans lequel le pH du mélange obtenu à l'étape (c) est maintenu à environ 7,5.

16. Procédé selon l'une quelconque des revendications 1 à 15 dans lequel le solide récupéré à l'étape (d) est rincé avec de l'eau et séché à une température allant jusqu'à 150°C avant d'être calciné.

17. Procédé selon l'une quelconque des revendications 1 à 16 dans lequel l'étape (e) est effectuée en chauffant le solide à une température allant de 475°C à 700°C pendant une période allant de 30 à 120 minutes.
